# NEUE EUROPÄISCHE PATENTSCHRIFT

(11) **EP 0 711 135 B2**
(45) Veröffentlichungstag und Bekanntmachung des Hinweises auf die Entscheidung über den Einspruch: **04.07.2007**
(45) Hinweis auf die Patenterteilung: 01.12.1999
(21) Anmeldenummer: 95922468.4
(22) Anmeldetag: 20.05.1995
(51) Int. Cl.: A61F 2/06

(54) **STENT**
STENT
EXTENSEUR

(30) Priorität: 26.05.1994 DE 4418336
(43) Veröffentlichungstag der Anmeldung: 15.05.1996
(73) Patentinhaber: Angiomed GmbH & Co. Medizintechnik KG, 76227 Karlsruhe (DE)
(72) Erfinder: LINDENBERG, Josef, A-9210 Pörtschach am Worthersee (AT); SCHNEPP-PRESCH, Wolfram, D-76185 Karlsruhe (DE)
(74) Vertreter: HOFFMANN EITLE
(86) Internationale Anmeldenummer: PCT/EP1995/001925
(87) Internationale Veröffentlichungsnummer: WO 1995/032688

(56) Entgegenhaltungen:
- EP-A- 0 540 290
- EP-A- 0 587 197
- EP-A- 0 647 438
- EP-A- 0 761 251
- WO-A-91/12779
- WO-A-93/17636
- WO-A-94/17754
- DE-A- 4 333 836
- GB-A- 2 189 150
- US-A- 4 994 066
- US-A- 5 064 435

## Beschreibung

Die Erfindung betrifft einen Stent zum Aufweiten und Offenhalten von Gefäßen, mit einem radial kontrahierten Zustand zum Einfuhren ins Gefäß und mit einem radial expandierten Zustand nach Einführen in das Gefäß, wobei der einteilige Stent Rippen und zwischen diesen Freiräume aufweist.

Derartige in einen Körperhohlraum, ein Gefäß oder dergleichen einbringbare Stents oder implantierbare Katheter können aus Kunststoff oder aus inertem Metall, wie Stahl oder Nickel- Titan-Legierungen, bestehen. Solche Stents werden insbesondere auch als endovaskuläre bzw. endoluminale Stents bzw. Endoprothesen bezeichnet. Die Stents werden beispielsweise zur Erweiterung des Harnleiters im Prostatabereich bei benigner Prostata-Hyperplasie (BPH) oder aber auch in verkalkten Blutgefäßen zur Erweiterung und Offenhaltung derselben eingesetzt Die Stents weisen Materialbereiche und Zwischenräume zwischen diesen auf. Hierdurch kann ein Umwachsen des Stents durch das Wandungsgewebe des offengehaltenen Organs erfolgen. Stents können spiralig oder in Form einer schraubenförmig gewundenen Wendel ausgebildet sein; sie können aus gewebtem oder gestricktem Draht- oder Kunststoffmaterial bestehen. Derartige Stents können Gedächtnis- oder Memory-Eigenschaften aufweisen, wie sie beispielsweise bei bestimmten Nickel- Titan-Legierungen (Nitinol) gegeben sind.

Ein gattungsgemäßer Stent ist aus der WO 94/17754 A1 bekannt.

Die US 5,064,435 zeigt einen zwei- oder mehrteilige.1 Stent zum Aufweiten und Offenhalten von Gefäßen mit einem radial kontrahierten Zustand zum Einführen in das Gefäß und mit einem radial expandierten Zustand nach Einführen in das Gefäß. Der Stent besteht aus mindestens zwei Stentteilen, die jeweils an einem Ende eine radiale Aufweitung besitzen. Die Stentteile sind mit ihrem nicht aufgeweiteten Ende ineinander geschoben und bilden eine Überlappung. Diese zweiteilige Ausbildung ist in fertigungstechnischer, aber auch in medizinischer Hinsicht als nachteilig anzusehen, weil das Gewebewachsverhalten im Bereich der Überlappung anders ist als im Bereich außerhalb derselben. Nachteilig ist darüber hinaus, daß die radialen Kräfte im Bereich der Aufweitung durch die gleichwertig ausgebildeten Rauten oder Zwischenräume fest vorgegeben sind.

Der Erfindung liegt die Aufgabe zugrunde, eine sichere Verankerung eines derartigen Stents im aufzuweitenden Gefäß zu ermöoglichen.

Erfindungsgemäß wird die genannte Aufgabe bei einem gattungsgemäßen Stent durch die Merkmale des Anspruchs 1 gelöst.

In bevorzugter Ausgestaltung ist dabei vorgesehen, daß die Länge der Rippen im Stirnbereich 120-190 % der Länge der Rippen im Hauptbereich des Stents beträgt.

Weitere bevorzugte Ausbildungen der Erfindung sehen vor, daß die Rippen in den Stirnseitenbereichen sich radial weiter nach außen erstrecken als die Rippen im Hauptbereich des Stents und daß die Rippen im Stirnseitenbereich einen endlichen Winkel zur Hauptachse des Stents einschließen.

Die Freiräume können entweder rauten- oder wabenförmig ausgebildet sein. Der Stent ist vorzugsweise selbstexpandierend und in bevorzugter Ausgestaltung nicht lediglich aufgrund elastischer Eigenschaften und Einführen in einem unter radialer Spannung stehenden Zustand, sondern dadurch, daß er aus Formgedächtnislegierung (Memory-Metall) besteht.

Um eine größere Biegefähigkeit und Flexibilität des Stents zu erreichen, sieht eine weitere bevorzugte Ausgestaltung vor, daß zwischen in axialer Richtung hintereinander angeordneten Rippen teils Zwischenräume, teils Verbindungsbereiche vorhanden sind. Hierdurch wird eine höhere Flexibilität erreicht, als es bei einem Stent der Fall wäre, bei dem in axialer Richtung aufeinanderfolgende Rippen in den Verbindungsbereichen fest miteinander verbunden sind. Es wird auch erreicht, daß bei Biegung unter Einwirkung achsensenkrechter Kräfte keine Querschnittsdeformation erfolgt.

Dadurch, daß der Stent einlagig ausgebildet ist, wird eine hohe Biegefähigkeit erreicht, ohne daß Metallkreuzungspunkte, wie dies bei Gestricken, Geflechten usw. der Fall ist, gegeben sind, die zu einer größeren Materialstärke führen. Es kann ein besseres Einwachsen des erfindungsgemäßen Stents ins Gewebe erfolgen. Weiterhin wird die Gefahr des Auftretens von Thrombosen, insbesondere im vaskulären Bereich, wesentlich reduziert bzw. praktisch ausgeschlossen.

In bevorzugter Ausgestaltung kann dabei vorgesehen sein, daß die Verbindungsbereiche in Umfangsrichtung zueinander versetzt sind. Hierdurch wird die gewünschte Axialfestigkeit (also gegen Stauchen und Zug in axialer Richtung) bei der Biegefestigkeit senkrecht zur Achse beibehalten bzw. erreicht.

Weitere bevorzugte Ausgestaltungen sehen vor, daß in dem Flachblech Schlitze zur Bildung der Zwischenräume herausgeschnitten sind, wobei das Flachblech nach Biegen zu einer Zylinderkontur in Randbereichen miteinander verbunden, insbesondere verschweißt ist und daß die Freiräume durch Schlitze nach Wärmebehandlung gebildet sind.

Weitere Vorteile und Merkmale der Erfindung ergeben sich aus den Ansprüchen und aus der nachfolgenden Beschreibung, in der ein bevorzugtes Ausführungsbeispiel der Erfindung unter Bezugnahme auf die Zeichnung im einzelnen erläutert ist. Dabei zeigt:
- Figur 1: eine bevorzugte Ausgestaltung des erfindungsgemäßen Stents in seinem radial kontrahierten Tieftemperatur- oder Einführzustand;
- Figur 2: den erfindungsgemäßen Stent in einem radial expandierten Hochtemperatur- oder Einsatzzustand; und
- Figur 3: eine sehr schematische Darstellung eines Teils eines Stents zur Verdeutlichung der Ausgestaltung an den stirnseitigen Enden.

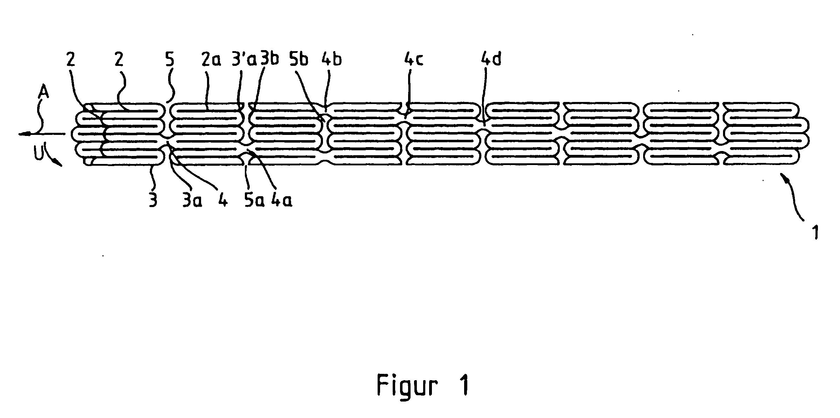

Der erfindungsgemäße Stent 1 weist in seinem radial kontrahierten Zustand zum Einführen in das aufzuweitende Gefäß eine zylindrische Form oder Außenkontur auf, wie sie in der Figur 1 dargestellt ist. Im expandierten Zustand weist der erfindungsgemäße Stent 1 über die Länge L seines Hauptkörpers, d.h. seine größte Länge, ebenfalls eine zylindrische Außenkontur auf; der erfindungsgemäße stent ist allerdings im Bereich seiner beiden Stirnseiten 1b, 1c über die Radialabmessungen, d.h. den Durchmesser D des Hauptkörpers 1a, radial aufgeweitet, so daß der Stent 1 sich mit seinen radial aufgeweiteten Enden 1b, 1c an der Gefäßwand verankern kann.

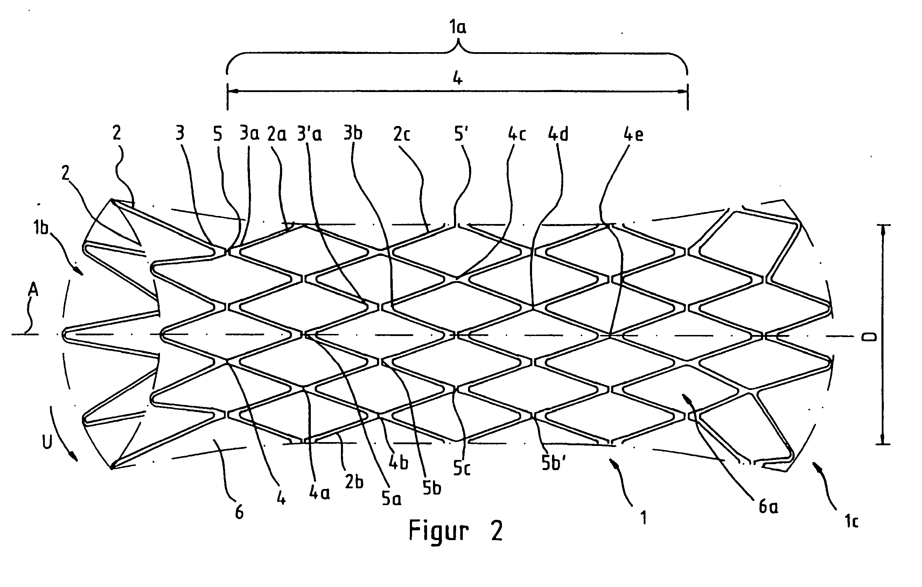

Der erfindungsgemäße Stent 1 besteht, wie insbesondere aus der Figur 2 deutlich wird, aus einer Reihe von in Achsrichtung A hintereinander angeordneten, über den Umfang des Stents 1 verlaufenden Mäanderbahnen in Form von bei Verbindungs- oder Spitzenbereichen 3, 3a, 3'a, 3b winklig miteinander verbundenen Rippen 2 bzw. 2a bzw. 2b. In Umfangsrichtung sind die Mäanderbahnen oder Rippen 2, 2a, 2b derart angeordnet, daß jeweils einander zugewandte, benachbarte Verbindungs- oder Spitzenbereiche 3, 3a bzw. 3'a, 3b von jeweils nebeneinander angeordneten Mäanderbahnen oder Rippen 2, 2a, 2b in Achsrichtung fluchten.

Der Figur 2 ist ebenfalls deutlich zu entnehmen, daß die in Achsrichtung aufeinanderfolgenden, jeweils durch die Rippen 2, 2a, 2b gebildeten Mäanderbahnen im Bereich ihrer Spitzen- oder Verbindungsbereiche 3, 3a, 3'a, 3b nicht durch Übergänge 4, 4a, 4b, 4c. 4d miteinander verbunden sind, sondern daß in Umfangsrichtung zwischen derartigen Übergängen 4-4d zweier benachbarter, durch die Rippen 2, 2a gebildeter Mäanderbahnen jeweils mehrere Lücken 5,5', 5a. 5b, 5b' usw. angeordnet sind. Die Übergangsbereiche 4-4d und Lücken 5-5b' werden gemeinsam auch als Knotenbereiche bezeichnet.

In einer anderen Betrachtungsweise kann der erfindungsgemäße Stent derart verstanden werden, daß er durch Rippen 2, 2a, 2b und zwischen diesen gebildeten Freiräumen 6, 6a gebildet ist, wobei die Freiräume 6, 6a im dargestellten Ausführungsbeispiel grundsätzlich die Kontur einer Raute haben, d.h. von vier Rippenbereichen begrenzt sind, grundsätzlich auch die Form einer Wabe haben können, wobei sie von sechs Rippenbereichen begrenzt sind. Auch bei dieser Betrachtungsweise sind die Knotenbereiche teilweise als Verbindungen 4-4d stehengelassen, während sie in anderen Bereichen durch die Lücken 5-5b' aufgeschnitten sind. Die Verbindungen oder Übergänge 4-4d fluchten in Achsrichtung nicht, sondern sind jeweils angular zueinander bzw. in Umfangsrichtung versetzt.

Durch die Lücken 5-5b' usw. wird eine hohe Flexibilität des erfindungsgemäßen Stents erreicht. Es wird insbesondere erreicht, daß der Stent 1 bei Biegung senkrecht zu seiner Langsachse A und damit Biegung der Längsachse selbst nicht im Mittelbereich derart einknickt, daß er seine im Querschnitt im wesentlichen kreisförmige Kontur verliert und in Einwirkrichtung der Kräfte in der Mitte flachgedrückt und senkrecht zur Einwirkrichtung der Kräfte etwa in der Mitte seiner Längserstreckung verbreitert wird, wie dies bei herkömmlichen Stents der Fall ist, bei denen sämtliche einander zugewandten, benachbarten Spitzen- oder Verbindungsbereiche 3, 3a etc. in Achsrichtung nebeneinander verlaufender Mäanderwindungen durch Verbindungshereiche oder Übergänge 4, 4a fest verbunden sind. Die Verbindungsbereiche oder Übergänge 4, 4a ... sind einstückig mit den sonstigen Teilen des Stents, insbesondere den Rippen 2, 2a ... und deren jeweils einander benachbarten Spitzen- oder Verbindungsbereichen 3, 3a ausgebildet.

Der Figur 1 ist zu entnehmen, daß die zwischen den Rippen 2, 2a etc. der Mäanderbahnen in der Hochtemperaturstellung ausgebildeten, im wesentlichen rautenförmigen Freiräume (Figur 2) in der Niedertemperatur- oder Einführstellung sich zu Schlitzen verjüngen und die Rippen 2 ... der Mäanderbahnen im wesentlichen parallel zueinander verlaufen.

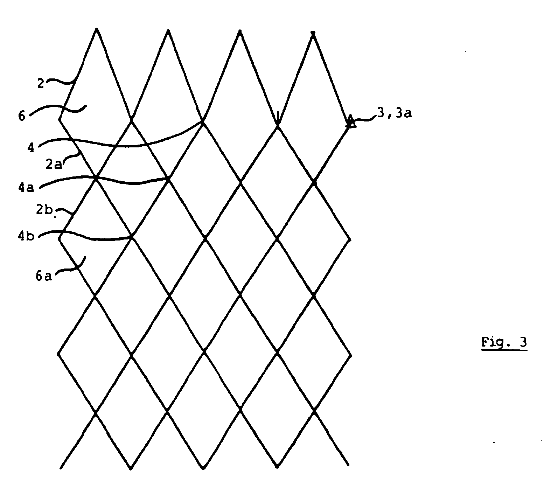

Insbesondere aus der Figur 3 ist entnehmbar, daß die Länge der Rippen 2 der äußeren oder stirnseitigen Mäanderhereiche deutlich größer ist als die Rippen 2a, 2b im Hauptkörper 1a des erfindungsgemäßen Stents 1. Die Länge der Endrippen 2 kann zwischen 120 und 200 % der Rippen 2a bis 2c des Hauptkörpers 1a betragen. Das Längenverhältnis ist weitgehend frei wählbar; es wird durch die zulässige Dehnung, die benötigte Stentlänge sowie die gewünschte radiale Aufweitung der Endbereiche 1b, 1c über den Hauptkörper 1a des Stents 1 hin bestimmt.

Der erfindungsgemäße Stent 1 besteht aus einer Nickel-Titan-Legierung, wie aus Nitinol. Er wird derart hergestellt, daß Blech zunächst mit hoher Genauigkeit, nämlich mit einem Toleranzbereich von 0,001 mm, auf die gewünschte Blechstärke geätzt wird. Anschließend werden die die Stents bildenden Teile aus großflächigem Blech ausgeschnitten. In diesen Blechteilen werden dann die Durchbrüche oder Schlitze derart eingeschnitten, daß in Umfangsrichtung benachbarte Schlitze jeweils um die Hälfte in ihrer Länge in Achsrichtung versetzt sind. Das Schneiden der Schlitze geschieht mittels eines Lasers. Im Mittelbereich jedes Schlitzes ist dieser mit einer Erweiterung versehen, so daß das die Erweiterung in Umfangsrichtung begrenzende Material etwa auf die Breite der zwischen den Schlitzen selbst verbliebenen Materialbestande reduziert wird. Diese Abschnitte bilden später, wenn sie stehengelassen werden, die Verbindungsabschnitte 4, 4'a, oder es werden in ihren Bereichen, wenn die Abschnitte entfernt werden, die Frei- oder Zwischenräume 5, 5a etc. geschaffen. Nach dem Schneiden der Schlitze erfolgt ein Ausbrechen der ausgeschnittenen Teile und ein Entgraten des Schlitzblechs. Anschließend wird das Schlitzblech zu einem Zylinder gebogen, so daß die seitlichen Rander sich berühren. Es werden dann an Laschen oder Ansätzen Verschweißungen vorgenommen, wodurch dann der Stent in seiner Tieftemperaturstellung entsprechend der Figur 1 entsteht Anschließend erfolgt eine Wärmebehandlung, um dem so geschaffenen Stent seine Gedächtniseigenschaften (Memory-Eigenschaften) zu verleihen, so daß er nach Temperaturerhöhung über eine vorgegebene Umgebungstemperatur, die deutlich unterhalb der Körpertemperatur des menschlichen Körpers liegt, sich in seine lochtemperaturstellung entsprechend der Figur 2 aufweiten kann, die er spätestens bei 35 °C erreicht hat.

Nachdem der Stent derart hergestellt und wärmebehandelt wurde, werden dann die Brücken in der gewünschten Weise entfernt, so daß die Verbindungsbereiche oder Stege 4, 4a bzw. die Freiräume 5, 5', 5a etc. gebildet werden, wie dies oben beschrieben wurde. Im folgenden erfolgt dann noch ein Schleifen und Polieren, vorzugsweise in einer Rotations-Trommelmaschine. Die Stents werden auf Maße. Funktion und Einstellung überprüft. Abschließend erfolgt ein Reinigen in einem Ultraschallbad, zunächst mit Seifenlösung, sodann mit destilliertem Wasser und schließlich mit Alkohol.

## Patentansprüche

1. Einteiliger Stent aus Flachblech, zum Aufweiten und Offenhalten von Gefäßen, mit einem radial kontrahierten Zustand zum Einführen ins Gefäß und mit einem radial expandierten Zustand nach dem Einführen in das Gefäß, wobei der Stent Rippen (2, 2a, 2b, 2c) und zwischen den Rippen Freiräume (6, 6a) aufweist, im radial expandierten Zustand mindestens ein stirnseitiges Ende (1b, 1c) eine größere Radialerstreckung aufweist als der restliche Hauptkörper (1a) des Stents, und Rippen (2) im Bereich zumindest eines Stirnendes (1b, 1c) des Stents (1) eine größere Länge aufweisen als entsprechende Rippen (2a, 2b, 2c) des Hauptkörpers (1a) des Stents (1).

2. Stent nach Anspruch 1, **dadurch gekennzeichnet, daß** die Länge der Rippen (2) im Stirnbereich (1b, 1c) 120 bis 190 % der Lange der Rippen im Hauptbereich (1a) des Stents (1) beträgt.

3. Stent nach einem der vorangehenden Ansprüche, **dadurch gekennzeichnet, daß** die Rippen in den Stirnseitenbereichen sich radial weiter nach außen erstrecken als die Rippen im Haupthereich des Stents.

4. Stent nach einem der Ansprüche 1 bis 3, **dadurch gekennzeichnet, daß** die Rippen (2) im Stirnseitenbereich. (1b, 1c) einen endlichen Winkel zur Hauptachse (A) des Stents (1) einschließen.

5. Stent nach einem der Ansprüche 1 bis 4, **dadurch gekennzeichnet, daß** die Zwischenräume (6, 6a) rautenförmig ausgebildet sind.

6. Stent nach einem der Ansprüche 1 bis 5, **dadurch gekennzeichnet, daß** die Zwischenräume wabenförmig ausgebildet sind.

7. Stent nach einem der vorangehenden Ansprüche, **dadurch gekennzeichnet, daß** er selbstexpandierend ist.

8. Stent nach einem der vorangehenden Ansprüche, **dadurch gekennzeichnet, daß** er aus einer Formgedächtnislegierung (Memory-Metall) besteht.

9. Stent nach einem der Ansprüche 1 bis 8, **dadurch gekennzeichnet, daß** zwischen in axialer Richtung hintereinander angeordneten Rippen teils Zwischenräume (5, ...), teils Verbindungshereiche (4, ...) vorhanden sind.

10. Stent nach Anspruch 11, **dadurch gekennzeichnet, daß** die Verbindungsbereiche (4, ...) in Umfangsrichtung zueinander versetzt sind.

11. Stent nach einem der vorangehenden Ansprüche, **dadurch gekennzeichnet, daß** in dem Flachblech Schlitze zur Bildung der Zwischenräume (6, 6a) herausgeschnitten sind, wobei das Flachblech nach Biegen zu einer Zylinderkontur in Randbereichen miteinander verbunden, insbesondere verschweißt ist.

## Claims

1. A one-piece stent of flat metal sheet for expanding and keeping open vessels, with a radially contracted state for insertion into the vessel and with a radially expanded state following insertion in the vessel, in which the stent has ribs (2, 2a, 2b, 2c) and between the ribs free spaces (6, 6a), in the radially expanded state at least one front end (1b, 1c) is of a greater radial extent than the remaining main body (1a) of the stent, and ribs (2) in the area of at least one front end (1b, 1c) of the stent (1) are of a greater length than corresponding ribs (2a, 2b, 2c) of the main body (1a) of the stent (1).

2. A stent according to claim 1 **characterised in that** the length of the ribs (2) in the front area (1b, 1c) is 120 to 190% of the length of the ribs in the main area (1a) of the stent (1).

3. A stent according to one of the preceding claims **characterised in that** the ribs in the front areas extend radially further outwardly than the ribs in the main area of the stent.

4. A stent according to one of claims 1 to 3 **characterised in that** the ribs (2) in the front area (1b, 1c) form a finite angle with the major axis (A) of the stent (1).

5. A stent according to one of claims 1 to 4 **characterised in that** the intermediate spaces (6, 6a) are of a rhombic shape.

6. A stent according to one of claims 1 to 5 **characterised in that** the intermediate spaces are of a honeycomb shape.

7. A stent according to one of the preceding claims **characterised in that** it is self-expanding.

8. A stent according to one of the preceding claims **characterised in that** it comprises a shape memory alloy (memory metal).

9. A stent according to one of claims 1 to 8 **characterised in that** between ribs arranged in succession in the axial direction there are in part intermediate spaces (5, ...) and in part connecting areas (4, ...).

10. A stent according to claim 11 **characterised in that** the connecting areas (4, ...) are mutually displaced in the circumferential direction.

11. A stent according to one of the preceding claims **characterised in that** slots are cut out in the flat metal sheet to form the intermediate spaces (6, 6a), wherein after bending to a cylindrical contour the flat metal sheet is connected together in edge regions, in particular welded.

## Revendications

1. Stent d'une seule pièce et réalisé dans une feuille de tôle pour dilater et maintenir ouverts des vaisseaux, présentant un état de contraction radiale pour son introduction dans le vaisseau et un état d'expansion radiale après son introduction dans le vaisseau, dans lequel le stent comporte des nervures (2,2a,2b,2c) et des espaces libres (6,6a) entre ces nervures, dans lequel à l'état expansé au moins une extrémité frontale (1b,1c) présente une extension radiale supérieure au reste du corps principal (1a) du stent, et dans lequel des nervures (2) situées dans la zone d'au moins une extrémité frontale (1b,1c) du stent (1) présentent une longueur supérieure à celle de nervures correspondantes (2a,2b,2c) du corps principal (1a) du stent (1).

2. Stent selon la revendication 1, **caractérisé en ce que** la longueur des nervures (2) dans la zone frontale (1b, 1c) correspond à 120 à 190 % de la longueur des nervures dans la zone principale (1a) du stent (1).

3. Stent selon l'une quelconque des revendications précédentes, **caractérisé en ce que** les nervures des zones frontales s'étendent radialement vers l'extérieur au-delà des nervures de la zone principale du stent.

4. Stent selon l'une quelconque des revendications 1 à 3, **caractérisé en ce que** les nervures (2) dans la zone de la face frontale (1b,1c) forment un angle de valeur finie par rapport à l'axe principal (A) du stent (1).

5. Stent selon l'une quelconque des revendications 1 à 4, **caractérisé en ce que** les espaces intermédiaires (6,6a) affectent une forme en losange.

6. Stent selon l'une quelconque des revendications 1 à 5, **caractérisé en ce que** les espaces intermédiaires sont alvéolaires.

7. Stent selon l'une quelconque des revendications précédentes, **caractérisé en ce qu'**il est autoexpansant.

8. Stent selon l'une quelconque des revendications précédentes, **caractérisé en ce qu'**il est réalisé dans un alliage à mémoire de forme (Memory-Metall).

9. Stent selon l'une quelconque des revendications 1 à 8, **caractérisé en ce qu'**entre des nervures alignées axialement se trouvent en partie des espaces intermédiaires (5,...), en partie des zones de liaison (4,...).

10. Stent selon la revendication 11, **caractérisé en ce que** les zones de liaison (4,...) sont décalées entre elles en périphérie.

11. Stent selon l'une quelconque des revendications précédentes, **caractérisé en ce que** dans la feuille de tôle sont découpées des fentes pour constituer les espaces intermédiaires (6,6a), la feuille de tôle étant solidarisée, notamment soudée dans ses zones d'extrémité après avoir été incurvée pour la conformer en cylindre.
